# EUROPEAN PATENT SPECIFICATION

(11) **EP 2 654 865 B1**
(45) Date of publication and mention of the grant of the patent: **25.02.2015**
(21) Application number: 11811154.1
(22) Date of filing: 20.12.2011
(51) Int. Cl.: A61M 11/06, F04C 18/12

(54) **NEBULIZER DEVICE**
VERNEBLER
DISPOSITIF NÉBULISEUR

(30) Priority: 22.12.2010 US 201061425835 P
(43) Date of publication of application: 30.10.2013
(73) Proprietor: Koninklijke Philips N.V., 5656 AE Eindhoven (NL)
(72) Inventor: ATTOLINI, Lorenzo, NL-5656 AE Eindhoven (NL)
(74) Representative: Steffen, Thomas
(86) International application number: PCT/IB2011/055802
(87) International publication number: WO 2012/085833

(56) References cited:
- EP-A2- 0 261 649
- US-A- 4 860 705
- US-A1- 2001 037 807
- US-A1- 2008 110 451
- US-B1- 6 732 731

## Description

The present invention relates to medication delivery apparatus, and in particular, to a nebulizer unit. The invention further relates to a nebulizer system having a compressor unit and a handset.

A number of devices are available for delivering a drug into the lungs of a patient. Once such device is a nebulizer, which is a device that is used for converting a liquid, such as a liquid medication, into an aerosol which is then inhaled by the patient, typically through a mouthpiece. A number of different types of nebulizers exist, such as, without limitation, jet nebulizers (sometimes referred to as pneumatic nebulizers) and ultrasonic nebulizers. A typical jet nebulizer uses compressed air to generate the aerosol from the liquid. The compressed air is typically provided by a compressor unit that produces a supply of compressed air through the reciprocating movement of a piston within a cylinder. Two examples of nebulizers that utilize piston compressor units are the MiniElite™ and MicroElite™ units manufactured and sold by the assignee of the present application, Philips Respironics.

Documents US 6732731 and EP0261649 disclose exemplary medical nebulizers comprising features of the preambles of appended claims 1 and 11 respectively.

There are a number of areas associated with piston-type compressor units in which improvements have been actively sought. One such area is in limiting the noise produced by such units as such noise can be irritating to both a user of the device as well as others in the general vicinity of a person using the device. The noise produced is largely caused by the movement of the compressor valves. The quick, flapping movement causes the valves to hit the compressor head and base thousands of times per minute, thus generating the rattling sound that is commonly associated with such units.

Another area of concern relates to vibrations produced by the unit. In common piston compressor units, vibrations are caused by the presence of an alternative mass (the piston and its components). The reciprocating movement of the piston creates constant vibrations which can cause a number of negative effects. In handheld units, such vibrations can make the unit uncomfortable to grasp for time periods required to complete a treatment. In nebulizer systems that utilize a stand-alone compressor unit and separate handset, the vibration of the compressor unit can cause excess noise as well as lead to potential instability of the compressor depending on its placement.

A further area of concern is the overall sizing of the nebulizer or nebulizer system. As such units are commonly portable, it is desirable to minimize the overall sizing of the nebulizer in such a manner that lends itself to portability.

While conventional nebulizers have been generally suitable for their intended uses there is still room for improvement in nebulizer devices, particularly in such units that employ compressors to provide a supply of compressed air.

Accordingly, it is an object of the present invention to provide a nebulizer for delivering a medication to a patient that overcomes the shortcomings of conventional nebulizers. This object is achieved according to one embodiment of the present invention by providing a nebulizer having a housing including: an inlet, a compressor, a reservoir and a nozzle assembly disposed therein. The compressor includes a number of rotor members, each rotor member being rotatable about a respective axis with each respective axis being parallel with every other respective axis and spaced an equal distance from a central axis of the compressor. The compressor further includes a compression chamber and a motor. The compression chamber has a variable volume defined by the relative positioning of the number of rotor members. The compression chamber is in selective fluid communication with the inlet. The motor has an output shaft operatively coupled to the number of rotor members in a manner such that rotation of the output shaft causes rotation of each rotor member of the number of rotor members. The motor is adapted to be coupled to a power source. The reservoir is adapted to receive a quantity of medication. The nozzle assembly is disposed adjacent the reservoir and in selective fluid communication with the compression chamber. The nozzle assembly is adapted to receive a flow of air from the compression chamber and utilize the flow to nebulize the quantity of medication contained in the reservoir for inhalation by a patient.

This object is achieved according to another embodiment of the present invention by providing a nebulizer system including a compressor unit, a handset adapted to receive a quantity of medication, and a conduit. The compressor unit includes a housing having an inlet, a compressor, a motor, and a port disposed therein or thereon. The compressor includes a number of rotor members, each rotor member being rotatable about a respective axis. Each respective axis is parallel with every other respective axis and spaced an equal distance from a central axis of the compressor. The compressor includes a compression chamber having a variable volume defined by the relative positioning of the number of rotor members. The compression chamber is in selective fluid communication with the inlet. The motor includes an output shaft operatively coupled to the number of rotor members in a manner such that rotation of the output shaft causes rotation of each rotor member of the number of rotor members. The motor is adapted to be coupled to a power source. The conduit includes a first end coupled to the port of the compressor unit and a second end coupled to the handset.
FIG. 1 is an isometric view of a particular embodiment of a nebulizer according to the principles of the present invention;
FIG. 2 is an elevational cross-sectional view of the nebulizer of FIG. 1;
FIG. 3 is a detail view of the portion 3-3 of the cross-sectional view of FIG. 2;
FIG. 4 is an elevational cross-sectional view of the nebulizer of FIG. 1 shown with the cap removed, a battery back installed, and a schematic mouthpiece installed in a treatment position;
FIG. 5 is a sectional view of the nebulizer of FIG. 1 taken along line 5-5 of FIG. 2;
FIG. 6 is a schematic view showing examples of the movement of the rotor members during compression and aspiration phases of a compressor unit according to the principles of the present invention;
FIG. 7 is partial schematic view a particular embodiment of a nebulizer system according to the principles of the present invention showing a cross-sectional view of a compressor unit schematically connected to a handset.

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality). As employed herein, the term "and/or" shall mean both alternatives (i.e., "and") or just one of the alternatives (i.e., "or").

Directional phrases used herein, such as, for example and without limitation, top, bottom, left, right, upper, lower, front, back, and derivatives thereof, relate to the orientation of the elements shown in the drawings and are not limiting upon the claims unless expressly recited therein. Like numbers refer to like elements throughout.

The present invention is directed to an improved nebulizer device that utilizes a 4-rotor compressor unit. Nebulizers constructed according to the principles described herein are more compact and offer quieter operation than conventional nebulizer devices.

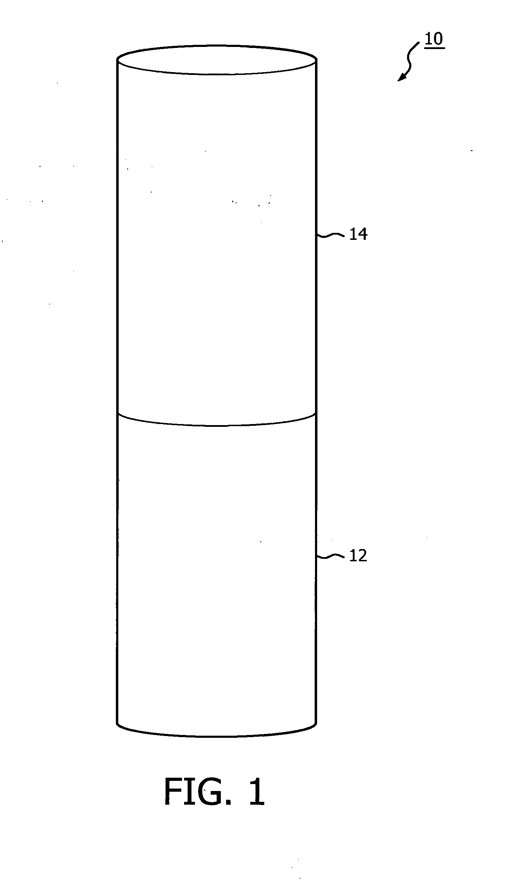

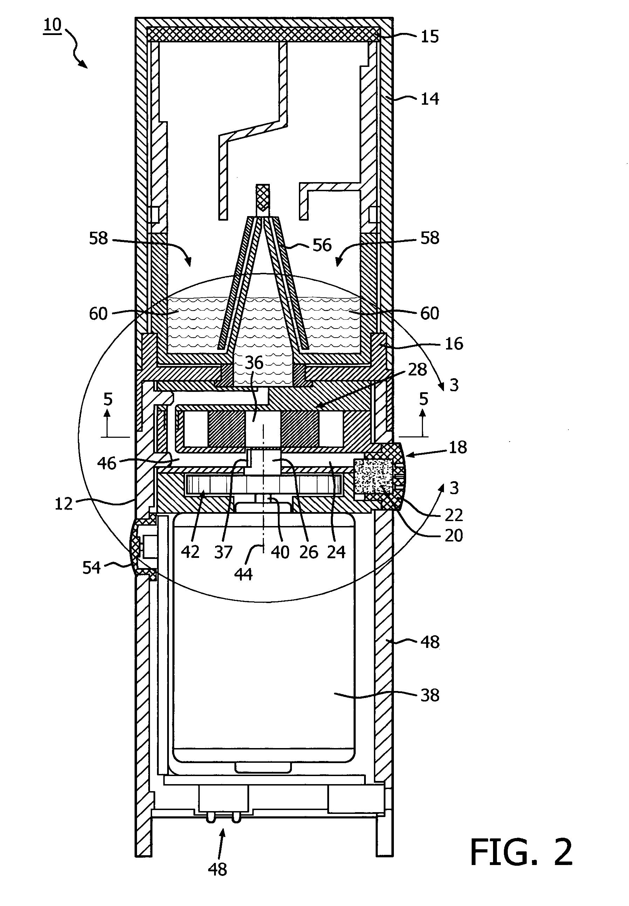

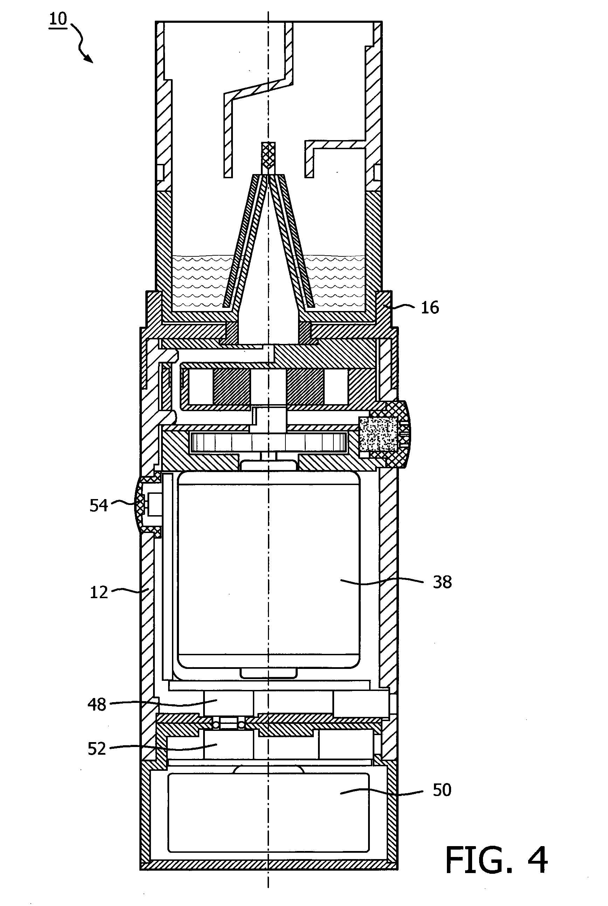

FIGS. 1-5 illustrate an exemplary embodiment of a nebulizer 10 and related accessories according to the principles of the present invention. Referring to FIG. 1, nebulizer 10 includes a housing 12 having a removable cap portion 14 selectively coupled to an upper portion 16 (FIGS. 2 and 4) of housing 12. Such coupling may be accomplished through a threaded engagement, slide fit, snap fit, or any other suitable coupling mechanism. Housing 12 and cap portion 14 are preferably formed from a rigid material (e.g., without limitation, metal, plastic) and formed in a generally cylindrical shape, however other shapes may be employed without varying from the scope of the present invention. Cap 14 is preferably provided with a seal member 15 (FIG. 2) formed from rubber or other suitable material that effectively seals housing 12 when cap portion 14 is installed thereon.

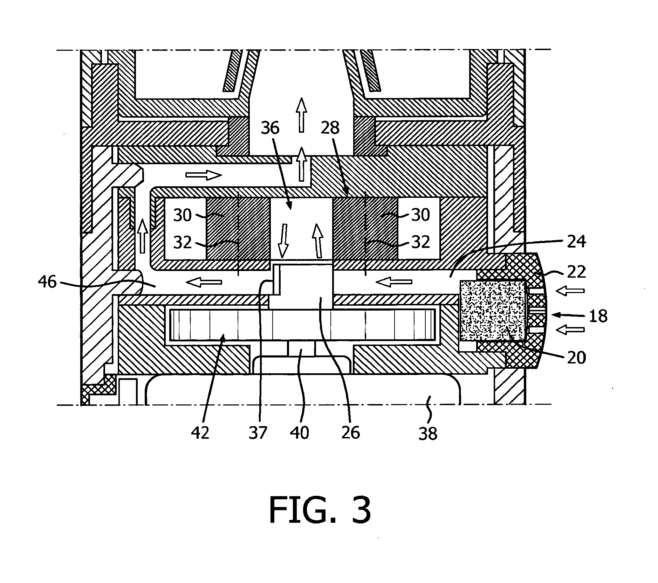

Referring to FIG. 2, housing 12 includes an inlet 18 through which air from the surrounding environment may enter, as shown by the arrows in FIG. 3. Inlet 18 includes a filter 20 and screen member 22. Filter 20 may be formed from any suitable material for preventing undesired contaminant particles from entering housing 12. A first conduit or passage 24 extends from inlet 18 inward toward a central shaft member 26 associated with a compressor 28.

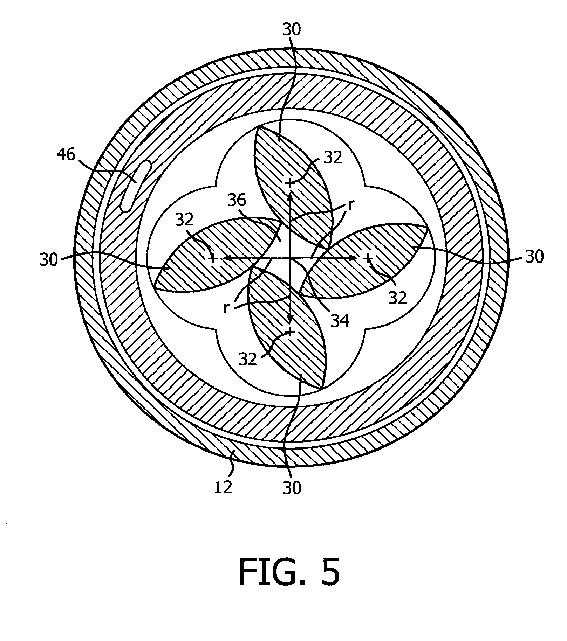

Compressor 28 is a rotary compressor mechanism of similar construction and operates using similar principles as those described in U.S. Patent Nos. 3,966,371 and 4,860,705 Compressor 28 includes a number of rotor members 30, each rotor member 30 being rotatable about a respective axis 32 (FIG. 3). Preferably, four rotor members 30 are used. Rotor members 30 are precisely formed from a rigid material, such as metal or other suitable material, and may be of an axially twisted shape (not shown) or have generally straight sidewalls and are typically oblong when viewed along respective axis 32, as shown in FIG. 5. Each respective axis 32 is oriented parallel to every other respective axis 32 and spaced an equal distance r (FIG. 5) from a central axis 34 of the compressor 28. A compression chamber 36 having a variable volume is defined by the relative positioning of the number of rotor members 30. Compression chamber 36 is a sealed volume aside from a channel 37 (FIGS. 2 and 3) provided in central shaft member 26 which functions as an inlet or outlet for compression chamber 36, depending on the positioning of central shaft member 26, as discussed in further detail below.

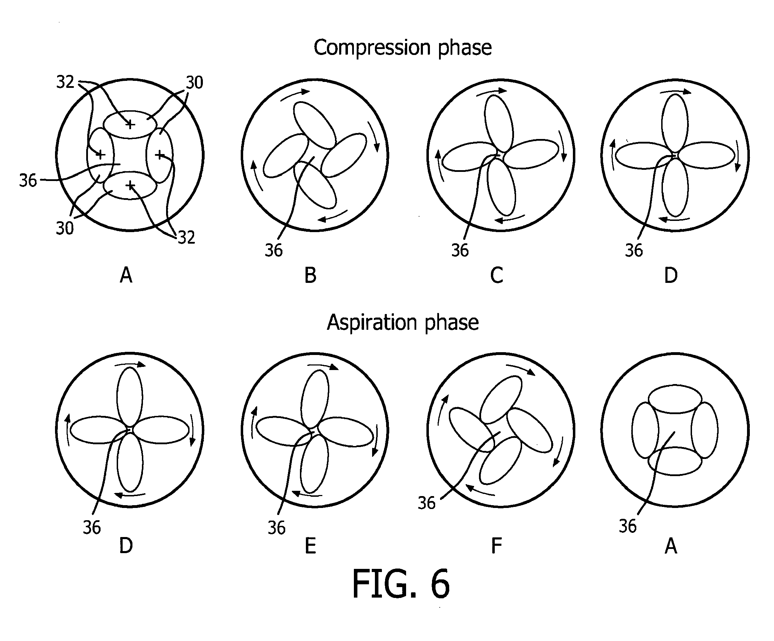

Having thus described the basic arrangement of compressor 28, operation thereof will now be described in conjunction with FIG. 6 which schematically depicts the varying volume of compression chamber 36 resulting from simultaneous clockwise rotation (shown by curved arrow) of rotor members 30 about respective axis 32 during a single compressor cycle. During the compression phase of a compressor cycle, the volume of compression chamber 36 is reduced, as shown by the progression of views A through D of FIG. 6, thus forcing air from compression chamber 36. Conversely, during the aspiration phase of a compression cycle, the volume of compression chamber 36 increases, as shown by the progression of views D through A of FIG. 6.

Rotation of rotor members 30 is caused by a motor 38 having an output shaft 40 operatively coupled to a respective shaft (not numbered) of each rotor member 30 by one or more gears of a gearset 42. Output shaft 40 is coupled to, or unitarily formed with, central shaft member 26 such that central shaft member 26 is also rotated by output shaft 40. In the depicted embodiments, output shaft 40 and central shaft member 26 are coupled in alignment and thus rotate along a single axis 44 (FIG. 2), which is also aligned with the central axis 34 of compressor 28. Channel 37 of central shaft member 26 is aligned such that during the compression phase of a compressor cycle, channel 37 is disposed such that air may exit compression chamber 36 and enter into a second conduit or passage 46 (as shown in FIGS 2 and 3). While in such positioning, the remainder of central shaft member 26 blocks off first passage 24. During the aspiration phase of a compressor cycle, channel 37 is disposed such that air may be drawn into compression chamber 36 from first passage 24. While in such positioning, the remainder of central shaft member 26 blocks off second passage 46. It is thus to be appreciated that central shaft member 26 functions as a valve/switch mechanism that controls air flow in and out of compression chamber 36. From such description it is to be appreciated that operation of compressor 28 by actuation of motor 38 thus produces a flow of air into housing 12 and through first and second passages 24 and 46 as shown by the shaded arrows in FIG. 3.

As shown in FIG. 2, motor 38 is preferably located in a lower portion 48 of housing 12 in order to provide for a lower overall center of gravity, and thus a more stable arrangement. Such positioning is also desirable to optimize space within, and thus the overall size of, housing 12. Continuing to refer to FIG. 2, motor 38 is adapted to be coupled to a power source through terminals 48. As shown in FIG. 4, such power source may, for example, without limitation, be a battery pack 50 having a set of terminals 52 that engage terminals 48 when battery pack 50 is coupled to the bottom (not numbered) of housing 12. In order to turn on and off motor 38 a switch member 54 or other suitable mechanism may be provided in housing 12.

Referring again to FIG. 2, second passage 46 continues from central shaft member 26 to a nozzle assembly 56 disposed adjacent a reservoir 58 near upper portion 16 of housing 12. Reservoir 58 is adapted to receive a quantity of medication 60 such as through the top of housing 12 when cap portion 14 is removed. For travel purposes, a treatment dosage of medication 60 may be deposited and stored in reservoir 58 with cap portion 14 coupled and sealed (via seal member 15) to housing 12. Nozzle assembly 56 may be of any suitable design for receiving a flow of air from the compression chamber 36 and utilizing such flow to nebulize the quantity of medication 60 for inhalation by a patient. Non-limiting examples of suitable nozzle assemblies are described in U.S. Patent No. 5,533,501.

FIG. 4 shows the nebulizer 10 with a mouthpiece 70 (shown schematically in hidden line) installed on and coupled to upper portion 16 of housing 12. Such coupling is preferably accomplished in a similar manner as cap portion 14 previously discussed (i.e., threaded engagement, slide fit, snap fit, or any other suitable coupling mechanism). Mouthpiece 70 is of conventional design, preferably formed from a lightweight plastic or other suitable rigid, or semi-rigid material. During a treatment, the patient would simply place mouthpiece 70 to their mouth, activate switch member 54 to turn on the nebulizer, and begin the treatment by inhaling nebulized particles of medication 60 produced by the nebulizer 10. Upon completing the treatment, the patient then simply shuts off the nebulizer, removes mouthpiece 70, and re-installs cap portion 14 in place of mouthpiece 70.

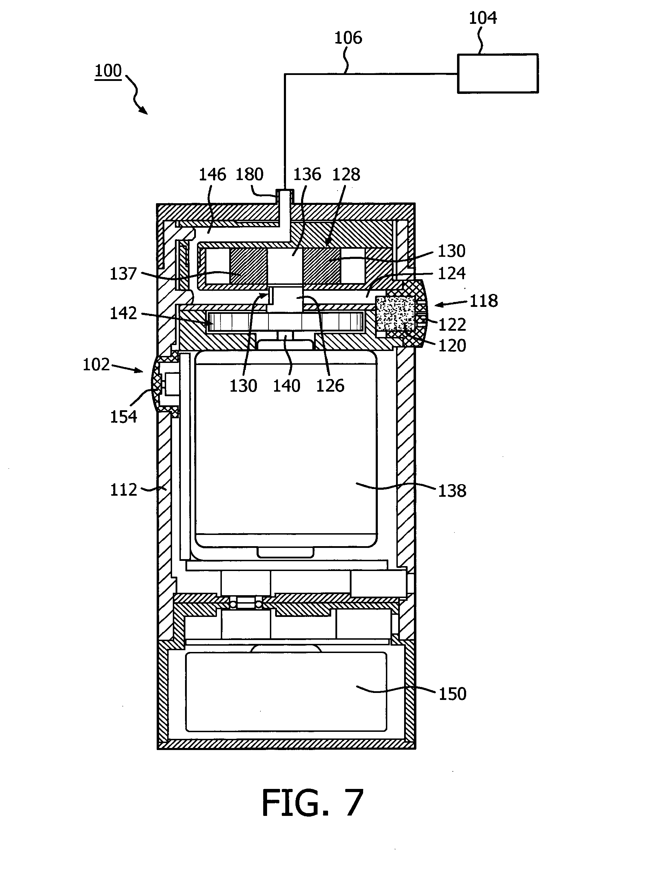

FIG. 7 shows an exemplary embodiment of a nebulizer system 100 according to the principles of the present invention. Nebulizer system 100 includes a compressor unit 102 and a handset 104 (shown schematically) coupled thereto via a conduit 106. Handset 104 may be any suitable handset capable of housing and nebulizing a quantity of medication when provided with a pressurized flow of air. Non-limiting examples of suitable handsets include those used with the MiniElite™ and MicroElite™ nebulizer systems manufactured and sold by the assignee of the present application, Philips Respironics. Conduit 106 is any device, such as flexible tubing, that carries the flow of gas from compressor unit 102 to handset 104. Similar to nebulizer 10, previously discussed, compressor unit 102 likewise includes, among other similar elements, a housing 112, an inlet 118, a filter 120, a screen member 122, a first conduit or passage 124, a central shaft member 126 having a channel 137, a compressor 128 having a number of rotor members 130, a compression chamber 136 having a variable volume defined by the relative positioning of the number of rotor members 130, a motor 138 having an output shaft 140, a gearset 142, a second conduit or passage 146, a battery pack 150, and a switch member 154. Such elements are generally arranged and function in a like manner as described in conjunction with nebulizer 10. Unlike nebulizer 10, however, compressor unit 102 includes a port 180 disposed on housing 112 which provides a flow of pressurized air to be utilized by handset 104 in nebulizing medication contained therein. Port 180 is situated to allow for conduit 106 to be readily coupled to compressor unit 102, thus completing nebulizer system 100.

In operation, a patient would simply connect handset 104 to port 180 of compressor unit 102 via conduit 106. The patient then would fill handset 104 with a required dosage of medication for inhalation, if not performed prior. The patient then would simply place handset 104 to their mouth and activate switch 154 of compressor unit 102 to begin the breathing treatment.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

## Claims

1. A nebulizer (10) comprising:
a housing (12);
an inlet (18) disposed in the housing;
a compressor (28) disposed in the housing;
a reservoir (58) disposed in the housing and adapted to receive a quantity of medication (60); and
a nozzle assembly (56) disposed in the housing adjacent the reservoir and in selective fluid communication with the compression chamber, the nozzle assembly adapted to receive a flow of air from the compression chamber and utilize the flow to nebulize the quantity of medication, **characterised in that** the compressor comprises:
a number of rotor members (30), each rotor member being rotatable about a respective axis (32), each respective axis being parallel with every other respective axis and spaced an equal distance (r) from a central axis (34) of the compressor;
a compression chamber (36) having a variable volume defined by the relative positioning of the number of rotor members, the compression chamber being in selective fluid communication with the inlet;
a motor (38) having an output shaft (40) operatively coupled to the number of rotor members in a manner such that rotation of the output shaft causes rotation of each rotor member of the number of rotor members, the motor being adapted to be coupled to a power source.

2. The nebulizer of claim 1 wherein the number of rotor members comprises four rotor members.

3. The nebulizer of claim 1 wherein each rotor member of the number of rotor members comprises a rotor member having generally straight sidewalls.

4. The nebulizer of claim 1 wherein each rotor member of the number of rotor members comprises a rotor member having an axially twisted shape.

5. The nebulizer of claim 1 wherein the output shaft of the motor rotates about an output axis (44) and wherein the output axis is aligned with the central axis (34) of the compressor.

6. The nebulizer of claim 1 wherein the housing is of a generally cylindrical shaped having a top end and an opposite bottom end.

7. The nebulizer of claim 6 wherein the housing comprises a removable cap (14) coupled proximate to the top end of the housing.

8. The nebulizer of claim 6 wherein the upper portion of the housing is adapted to be coupled to a mouthpiece.

9. The nebulizer of claim 6 wherein the housing comprises a battery pack (50) disposed therein proximate the bottom end, the battery pack being electrically coupled to motor.

10. The nebulizer of claim 9 wherein the motor is disposed adjacent to, and above, the battery pack.

11. A nebulizer system (100) comprising:
a compressor unit (102) comprising:
a housing (112);
an inlet (118) disposed in the housing;
a compressor (128) disposed in the housing; and
a port (180) disposed on the housing;
a handset (104) adapted to receive a quantity of medication; and
a conduit (106) having a first end coupled to the port of the compressor unit and a second end coupled to the handset,
**characterised in that**
the compressor unit (102) comprises:
the compressor has a number of rotor members (130), each rotor member being rotatable about a respective axis, each respective axis being parallel with every other respective axis and spaced an equal distance from a central axis of the compressor; and
a compression chamber (136) having a variable volume defined by the relative positioning of the number of rotor members, the compression chamber being in selective fluid communication with the inlet;
a motor (138) disposed on the housing, the motor having an output shaft (140) operatively coupled to the number of rotor members in a manner such that rotation of the output shaft causes rotation of each rotor member of the number of rotor members, the motor being adapted to be coupled to a power source.

## Patentansprüche

1. Zerstäuber (10), umfassend:
ein Gehäuse (12);
einen Einlauf (18), der im Gehäuse angeordnet ist;
einen Kompressor (28), der im Gehäuse angeordnet ist;
einen Behälter (58), der im Gehäuse angeordnet und angepasst ist, um eine Menge an Arzneimittel (60) aufzunehmen; und
eine Düsenbaugruppe (56), die im Gehäuse, an den Behälter angrenzend und in einer selektiven Fluidverbindung mit der Kompressionskammer angeordnet ist, wobei die Düsenbaugruppe angepasst ist, um eine Luftströmung aus der Kompressionskammer zu empfangen, und die Strömung zu verwenden, um die Menge an Arzneimittel zu zerstäuben, **dadurch gekennzeichnet, dass** der Kompressor folgendes umfasst:
eine Anzahl an Rotorelementen (30), wobei jedes Rotorelement um eine jeweilige Achse (32) drehbar ist, und jede jeweilige Achse parallel zu jeder anderen jeweiligen Achse, und in einem gleichen Abstand (r) von einer zentralen Achse (34) des Kompressors angeordnet ist;
eine Kompressionskammer (36), die ein variables Volumen aufweist, das durch die Positionierung der Anzahl an Rotorelementen zueinander definiert wird, wobei sich die Kompressionskammer in einer selektiven Fluidverbindung mit dem Einlass befindet;
einen Motor (38) mit einer Auslasswelle (40), die dergestalt wirkend an die Anzahl an Rotorelementen gekoppelt ist, dass die Drehung der Auslasswelle die Drehung eines jeden Rotorelements der Anzahl an Rotorelementen bewirkt, wobei der Motor angepasst ist, um an eine Energiequelle gekoppelt zu werden.

2. Zerstäuber nach Anspruch 1, bei dem die Anzahl an Rotorelementen vier Rotorelemente umfasst.

3. Zerstäuber nach Anspruch 1, bei dem jedes Rotorelement der Anzahl an Rotorelementen ein Rotorelement mit allgemein geraden Seitenwänden umfasst.

4. Zerstäuber nach Anspruch 1, bei dem jedes Rotorelement der Anzahl an Rotorelementen ein Rotorelement mit einer axial verdrillten Form umfasst.

5. Zerstäuber nach Anspruch 1, bei dem sich die Auslasswelle des Motors um eine Auslassachse (44) dreht, und bei dem die Auslassachse auf die zentrale Achse (34) des Kompressors ausgerichtet ist.

6. Zerstäuber nach Anspruch 1, bei dem das Gehäuse eine allgemein zylindrische Form mit einem oberen und einem gegenüberliegenden unteren Ende aufweist.

7. Zerstäuber nach Anspruch 6, bei dem das Gehäuse einen abnehmbaren Deckel (14) umfasst, der nahe am oberen Ende des Gehäuses angekoppelt ist.

8. Zerstäuber nach Anspruch 6, bei dem der obere Abschnitt des Gehäuses angepasst ist, um an ein Mundstück gekoppelt zu werden.

9. Zerstäuber nach Anspruch 6, bei dem das Gehäuse einen Batteriepack (50) umfasst, der dort in der Nähe des unteren Endes angeordnet ist, wobei der Batteriepack elektrisch mit dem Motor verbunden ist.

10. Zerstäuber nach Anspruch 9, bei dem der Motor angrenzend an, und über dem Batteriepack angeordnet ist.

11. Zerstäubersystem (100), umfassend:
eine Kompressoreinheit (102), umfassend:
ein Gehäuse (112);
einen Einlauf (118), der im Gehäuse angeordnet ist;
einen Kompressor (128), der im Gehäuse angeordnet ist; und
einen Port (180), der am Gehäuse angeordnet ist;
einen Handapparat (104), der angepasst ist, um eine Menge an Arzneimittel zu empfangen; und
einen Kanal (106) mit einem ersten Ende, das an den Port der Kompressoreinheit gekoppelt ist, und einem zweiten Ende, das an den Handapparat gekoppelt ist,
**dadurch gekennzeichnet, dass**
die Kompressoreinheit (102) folgendes umfasst:
eine Kompressionskammer (136) mit einem variablen Volumen, das durch die jeweilige Positionierung der Anzahl an Rotorelementen zueinander definiert wird, wobei sich die Kompressionskammer in einer selektiven Fluidverbindung mit dem Einlass befindet;
einen Motor (138), der am Gehäuse angeordnet ist, wobei der Motor über eine Auslasswelle (140) verfügt, die dergestalt wirkend an die Anzahl an Rotorelementen gekoppelt ist, dass die Drehung der Auslasswelle die Drehung eines jeden Rotorelements der Anzahl an Rotorelementen bewirkt, wobei der Motor angepasst ist, um an eine Energiequelle gekoppelt zu werden, und
der Kompressor über eine Anzahl an Rotorelementen (130) verfügt, wobei jedes Rotorelement um eine jeweilige Achse drehbar ist, und jede jeweilige Achse parallel zu jeder anderen jeweiligen Achse, und in einem gleichen Abstand von einer zentralen Achse des Kompressors angeordnet ist.

## Revendications

1. Nébuliseur (10) comprenant :
un logement (12) ;
une entrée (18) disposée dans le logement ;
un compresseur (28) disposé dans le logement ;
un réservoir (58) disposé dans le logement et apte à recevoir une quantité de médicament (60) ; et
un assemblage de buse (56) disposé dans le logement adjacent au réservoir et en communication fluidique sélective avec la chambre de compression, l'assemblage de buse étant apte à recevoir un flux d'air provenant de la chambre de compression et à utiliser le flux pour nébuliser la quantité de médicament,
**caractérisé en ce que** le compresseur comprend :
un nombre d'organes de rotor (30), chaque organe de rotor étant rotatif autour d'un axe respectif (32), chaque axe rotatif étant parallèle à chaque autre axe respectif et espacé d'une distance égale (r) d'un axe central (34) du compresseur ;
une chambre de compression (36) comportant un volume variable défini par le positionnement relatif du nombre d'organes de rotor, la chambre de compression étant en communication fluidique sélective avec l'entrée ;
un moteur (38) comportant un arbre de sortie (40) couplé de manière opérationnelle au nombre d'organes de rotor de telle sorte qu'une rotation de l'arbre de sortie provoque une rotation de chaque organe de rotor du nombre d'organes de rotor, le moteur étant apte à être couplé à une source d'alimentation électrique.

2. Nébuliseur selon la revendication 1, dans lequel le nombre d'organes de rotor comprend quatre organes de rotor.

3. Nébuliseur selon la revendication 1, dans lequel chaque organe de rotor du nombre d'organes de rotor comprend un organe de rotor ayant des parois latérales généralement droites.

4. Nébuliseur selon la revendication 1, dans lequel chaque organe de rotor du nombre d'organes de rotor comprend un organe de rotor ayant une forme axialement vrillée.

5. Nébuliseur selon la revendication 1, dans lequel l'arbre de sortie du moteur tourne autour d'un axe de sortie (44) et dans lequel l'axe de sortie est aligné avec l'axe central (34) du compresseur.

6. Nébuliseur selon la revendication 1, dans lequel le logement est de forme généralement cylindrique comportant une extrémité supérieure et une extrémité inférieure opposée.

7. Nébuliseur selon la revendication 6, dans lequel le logement comprend un capuchon amovible (14) couplé à proximité de l'extrémité supérieure du logement.

8. Nébuliseur selon la revendication 6, dans lequel la portion supérieure du logement est apte à être couplée à un embout buccal.

9. Nébuliseur selon la revendication 6, dans lequel le logement comprend un bloc-batterie (50) disposé à l'intérieur de celui-ci à proximité de l'extrémité inférieure, le bloc-batterie étant électriquement couplé au moteur.

10. Nébuliseur selon la revendication 9, dans lequel le moteur est disposé adjacent au bloc-batterie et au-dessus de celui-ci.

11. Système de nébuliseur (100) comprenant :
une unité de compresseur (102) comprenant :
un logement (112) ;
une entrée (118) disposée dans le logement ;
un compresseur (128) disposé dans le logement ; et
un orifice (180) disposé sur le logement ;
un combiné (104) apte à recevoir une quantité de médicament ; et
un conduit (106) ayant une première extrémité couplée à l'orifice de l'unité de compresseur et une deuxième extrémité couplée au combiné,
**caractérisé en ce que**
l'unité de compresseur (102) comprend :
une chambre de compression (136) comportant un volume variable défini par le positionnement relatif du nombre d'organes de rotor, la chambre de compression étant en communication fluidique sélective avec l'entrée ;
un moteur (138) disposé sur le logement, le moteur comportant un arbre de sortie (140) couplé de manière opérationnelle au nombre d'organes de rotor de telle sorte qu'une rotation de l'arbre de sortie provoque une rotation de chaque organe de rotor du nombre d'organes de rotor, le moteur étant apte à être couplé à une source d'alimentation électrique ; et
le compresseur comprend un nombre d'organes de rotor (130), chaque organe de rotor étant rotatif autour d'un axe respectif, chaque axe rotatif étant parallèle à chaque autre axe respectif et espacé d'une distance égale d'un axe central du compresseur.
